# EUROPÄISCHE PATENTSCHRIFT

(11) **EP 0 373 352 B1**
(45) Veröffentlichungstag und Bekanntmachung des Hinweises auf die Patenterteilung: **31.01.1996**
(21) Anmeldenummer: 89120548.6
(22) Anmeldetag: 07.11.1989
(51) Int. Cl.: C12Q 1/70, C12Q 1/68

(54) **Verfahren zum Nachweis von gesplei ter mRNA**
Method of detecting spliced mRNA
Méthode pour détecter de l'ARNm épissée

(30) Priorität: 11.11.1988 DE 3838269
(43) Veröffentlichungstag der Anmeldung: 20.06.1990
(73) Patentinhaber: BEHRINGWERKE Aktiengesellschaft, D-35001 Marburg (DE)
(72) Erfinder: Cerutti, Peter, Dr., CH-1012 Pully (CH); Whitcomb, Jeannette, Dr., Gettysburg, Pa 17325 (US); Zijlstra, Jacob, Dr., CH-1296 Coppet (CH); De Villiers, Ethel-Michelle, Dr., D-6945 Hirschberg (DE)

(56) Entgegenhaltungen:
- EP-A- 0 237 362
- EP-A- 0 256 321
- WO-A-88/06634
- J. EXP. MED., Band 167, Nr. 1, Januar 1988, Seiten 225-230, The Rockefeller University Press; D.K. SHIBATA et al.
- NATURE, Band 314, 7. März 1985, Seiten 111-114; E. SCHWARZ et al.
- PROC. NATL. ACAD. SCI. USA, Band 83, Juli 1986, Seiten 4680-4684; D. SMOTKIN et al.
- NATURE, Band 335, Nr. 6193, 27. Oktober 1988, Seiten 765-766, London, GB; D.J. McCANCE;
- CANCER RESEARCH, Band 48, 1. Juni 1988, Seiten 2969-2974; A. SCHNEIDER-GÄDICKE et al.

## Beschreibung

Die Erfindung betrifft ein PCR-Verfahren zum Nachweis von gespleißter mRNA unter Verwendung von Oligonukleotid-Primern. Dabei wurde die vor kurzem entwickelte PCR (Saiki et al. (1988) Science 239, 487-491) modifiziert. Speziellere PCR-Verfahren zum Nachweis von für bestimmte Typen des Humanen Papillomavirus spezifische DNA wurden bereits in der WO-A-8 806 634 sowie in Shibata et al., J. Exp. Med. 167, No. 1, 225-230 (1988) beschrieben. Die teilweise Charakterisierung des Genoms und der cDNA von HPV18 wird in der Europäischen Patentanmeldung 0 256 321 offenbart. In Cancer Research, Vol. 48, Seiten 2969-2974, (1.6. 1988), sind unter anderem HPV18-E6* Spleißsequenzen beschrieben.

Wir haben gefunden, daß durch Auswahl geeigneter Oligonukleotid-Primer (Amplimere), welche eine Spleißregion überspannen, und indem ein cDNA-Syntheseschritt mit reverser Transskriptase sowie eine PCR unter Verwendung besagter Amplimere nacheinander durchgeführt werden, gespleißte RNA identifiziert und soweit amplifiziert werden kann, so daß ein nichtisotopischer Nachweis möglich ist. Besonders vorteilhaft kann das erfindungsgemäße Verfahren zum direkten Nachweis von gespleißter HPV-mRNA eingesetzt werden. Dies gibt einen Hinweis auf prämaligne oder maligne Zustände bzw. Läsionen.

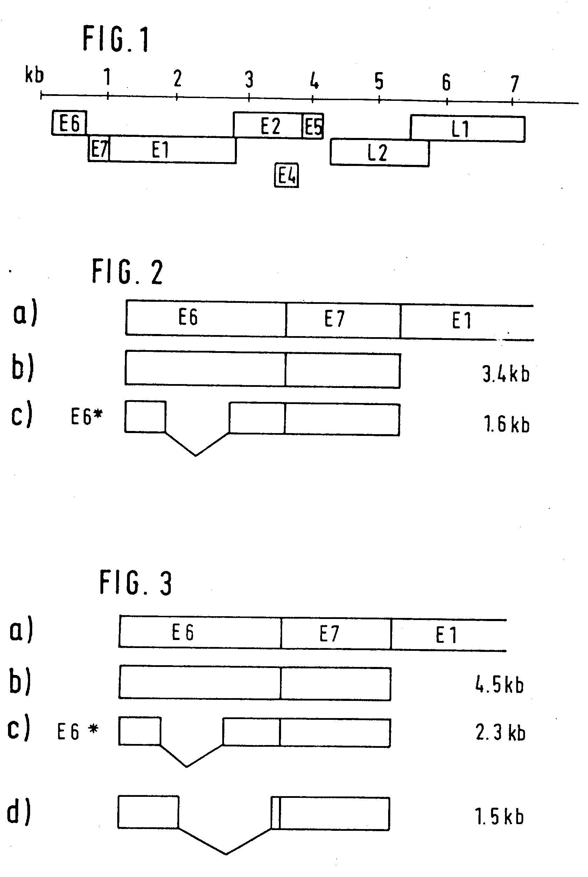

Zwischen dem Auftreten von Zervix-Karzinomen und dem Nachweis von HPV Serotypen 16, 18, 31 und 33 in Zervikalgewebe besteht eine starke Korrelation. Die verwandten HPV Serotypen 6 und 11 sind im Genitaltrakt zwar auch oft vorhanden, aber mit benignen Läsionen, Genitalwarzen und Kondylomata assoziiert. Das Genom von HPV 16 und 18 besitzt 8 offene Leserahmen ("Open Reading Frames", ORF; Fig. 1). L-1 und L-2 kodieren für Strukturprotein, während die Bedeutung der anderen ORF nicht so gut verstanden wird. Es gibt einen starken Zusammenhang zwischen in das Wirtsgenom integrierten E6/E7-Regionen und der Transformation zu Malignität. Für den E7 ORF wurde gezeigt, daß er für ein in HPV-infizierten Zellen in großen Mengen vorhandenes zytoplasmatisches Phosphoprotein kodiert. Die Rolle dieses Proteins für die Transformation und Aufrechterhaltung der Malignität ist nicht bekannt. Die E6/E7-Regionen werden in transformierten Zellen stark transkribiert. Es existiert sowohl ein Transkript der gesamten E6/E7-Region als auch gespleißte RNA's. Das in HPV 16 und 18 ähnliche Spleißmuster führt zu einem Translationsprodukt, das als E6^{*} bezeichnet wird. (Schneider-Gädicke et al. (1988) Cancer Res. 48, 2969-2974). Dieses Spleißmuster korreliert wahrscheinlich mit dem malignen Potential der HPV Viren, wie HPV 16, 18, 31 und 33 es aufweisen, während HPV 6 und 11 es nicht besitzen. Das gespleißte und im Leserahmen verschobene mRNA Transkript für HPV 16 bzw. HPV 18 E6^{*} ist schematisch in Fig. 2 und Fig.3 dargestellt. Zusätzlich ist eine für HPV 16 gefundene, noch kleinere gespleißte mRNA von etwa 1.5 kb gezeigt. Die bisher zur Verfügung stehenden Methoden zum Virus-Nachweis, wie beispielsweise in situ DNA-Hybridisierung mit radioaktiv markierten "DNA-Probes" sind schwierig durchzuführen, zeitaufwendig und oft nicht empfindlich genug. Das zur Amplifikation von HPV-DNA benutzte Verfahren der Polymerase Kettenreaktion (PCR; Saiki et al. a.a.O.) war gleichfalls nicht für die Routine geeignet.

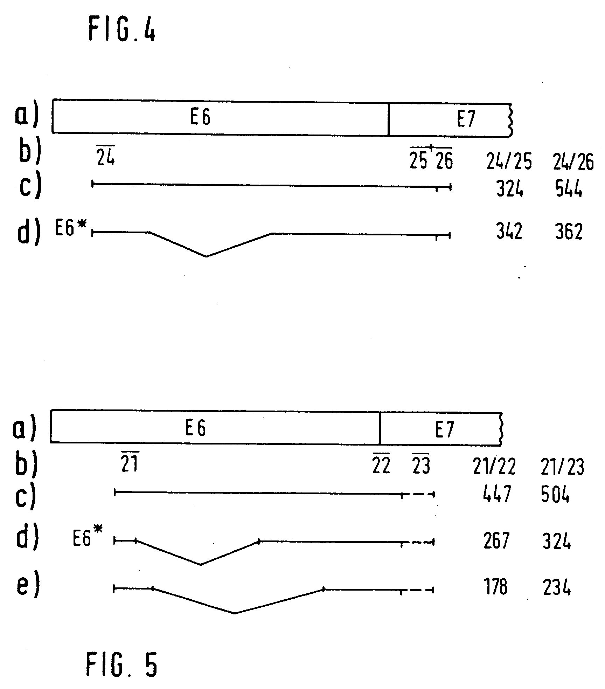

Wir haben gefunden, daß zum Nachweis von HPV die Polymerasekettenreaktion vereinfacht durchgeführt werden kann und bei Einsatz geeigneter Amplimere und darauf abgestimmter Dimethylsulfoxid-Konzentrationen HPV nach etwa 3 Stunden aus Zervix-Abstrichen nachweisbar ist. Dabei wurde als Polymerase die Taq-Polymerase eingesetzt und Temperaturen von 89 °C und 63 °C bei Zykluszeiten von 1 Minute gewählt. Bei Vorschaltung eines cDNA-Syntheseschrittes mit Hilfe von reverser Transkriptase kann anhand der auftretenden DNA-Banden auf das Vorliegen von gespleißter mRNA zu E6^{*} geschlossen werden (Fig. 4 und Fig.5) und damit das Vorliegen von prämalignen bzw. malignen Läsionen wahrscheinlich gemacht werden.

Die Erfindung betrifft folglich:
a) ein Verfahren zum Nachweis gespleißter RNA, dadurch gekennzeichnet, daß ein cDNA-Syntheseschritt mit reverser Transskriptase und eine PCR unter Verwendung von Amplimeren, welche eine Spleißregion überspannen, nacheinander durchgeführt werden;
b) wobei eine geeignete Wahl von Amplimeren den simultanen Nachweis von RNA mehrerer Virustypen (z.B. HPV 16, 18, 31 und 33) erlaubt und DNA-Fragmente von charakteristischer Länge für jedes einzelne Virus erhalten werden, die nach Agaroseelektrophoresetrennung und Anfärbung mit Ethidiumbromid densitomertisch quantifiziert werden;
c) wobei die Amplimere bevorzugt eine Spleißregion der HPV 16 oder HPV 18 E6-Region überspannen;
d) wobei die nachzuweisende mRNA durch Beschallung aus einer Zellprobe freigesetzt werden kann;
e) wobei die PCR bei Zugabe von bis zu 20% v/v Dimethylsulfoxid durchgeführt werden kann;
f) wobei die PCR unter Einsatz von Deoxynukleotidtriphosphatkonzentrationen >0,2 mM und Amplimerkonzentrationen >0,5 µM, mit Zykluszeiten von 1 bis 2 Minuten, bei zwei Temperaturen von 70°C bis 95°C und 50°C bis 70°C, bevorzugt bei den Temperaturen 89°C und 63°C, nach Start der PCR oberhalb der Schmelztemperatur und mit direktem Nachweis spezifischer DNA-Fragmente durch Färbung nach Agaroseelektrophorese durchgeführt wird, so daß die Spezifität wesentlich verbessert ist;
g) ein Verfahren zum Nachweis prämaligner oder maligner Zustände oder Läsionen durch den direkten Nachweis gespleißter HPV-spezifischer mRNA gemäß Punkt b).

Zur internen Standardisierung und zur Kontrolle, daß die Amplifikationsreaktionen zufriedenstellend funktioniert haben, kann ein "Single-copy"-Gen, wie beispielsweise das humane IL-2-Rezeptor-Gen, das β-Globin-Gen oder das c-H-ras-Gen koamplifiziert werden.

Es können Amplimere von 14 - 20 Nukleotiden gewählt werden, die etwa symmetrisch die Spleißstelle überspannen, falls nur Amplifikationsprodukte der Reversen Transkriptasereaktion erhalten werden sollen. Ein Beispiel ist der Amplimer 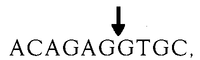
ACAGAGGTGC,
 wobei der Pfeil die Spleißstelle markiert. Die E6^{*} RNA wird jedoch auch durch ihre unterschiedliche Fragmentlänge nachgewiesen, wie in den Beispielen mit den amplimeren Paaren 24/26 und 21/22 für HPV 18 bzw. HPV 16 ausgeführt.

Die Erfindung ist ferner in den nachfolgenden Beispielen und Patentansprüchen beschrieben.

### Beispiel 1:

### Probennahme klinischer Spezimen zum HPV-DNA Nachweis und/oder E6^{*} RNA Nachweis

Nach der Applikation des Zervix-Abstrichs für die histologische Untersuchung ("pap smear") wurde der übliche Holzspatel, der das restliche Material enthält, in 20 ml eiskalte Earle's BSS-Glukose eingetaucht (0.2 g/l CaCl₂, 0.4 g/l KCL, 0.2 g/l MgSO₄, 7 H₂0, 6.8 g/l NaCl, 2.2 g/l NaHCO₃, 0.14 g/l NaH₂PO₄, H₂O, 1 g/l Glukose). Nach max. 5 Std. auf Eis wurde die Probe von Hand geschüttelt und der Spatel entfernt. Die Probe wurde bei 4 °C/100 x g zentrifugiert, wobei der Mucus oben aufschwimmt, während die Zellen sich auf dem Grund des Röhrchens absetzen. Der Überstand wurde dekantiert und der Zell-Rückstand einmal mit phosphatgepufferter Salzlösung gewaschen. Der Zell-Rückstand wurde bei -70 °C gefroren und bis zur weiteren Verarbeitung gefroren aufbewahrt.

### Beispiel 2:

### Reverse Transkription

Ein Reaktionszusatz von 5 µl Gesamtvolumen ist wie folgt zusammengesetzt:

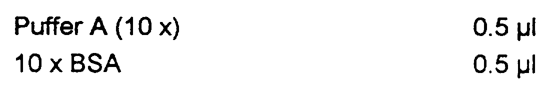

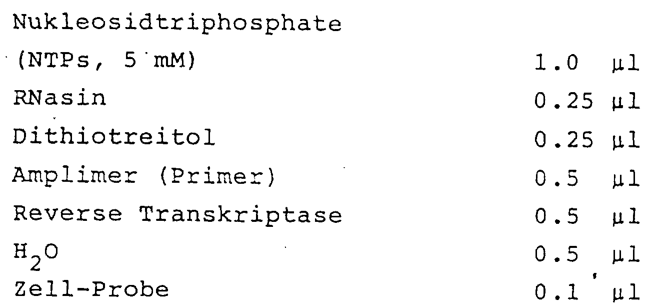

Puffer A (10x) ist dabei wie folgt zusammengesetzt:

| | |
|---|---|
| 500 mM | Tris HCl pH 8.3 |
| 70 mM | MgCl₂ |
| 500 mM | KCl |
| 100 mM | β-mercaptoethanol |

10 x BSA enthält 1.70 mg/ml BSA in H₂O RNasin (Promega) enthält 40 U/µl
Dithiothreitol ist 20 mM
Amplimere sind in H₂O in einer Konzentration von 400 µg/ml enthalten. Reverse Transkriptase (Boehringer Mannheim) enthält 20 - 25 U/µl.

Der Reaktionsansatz ohne Zugabe der Zellprobe wurde gemischt und per 4 µl im Eppendorfröhrchen pipettiert; darauf fügte man je 1 µl der zu untersuchenden Zellprobe hinzu. Anschließend wurde mit 200 µl Paraffinöl überschichtet, der Ansatz 15 Sekunden auf Stufe 2 mit einem Bronson Ultraschallgerät B15 beschallt und die Emulsion bei Raumtemperatur in der Eppendorf-Tischzentrifuge getrennt. Schließlich folgte eine Inkubation bei 42°C für 10 Minuten mit anschließendem Reaktionsstop durch Erhitzen auf 89°C.

### Beispiel 3: Amplifikation

Ein Reaktionsansatz ist aus 20 µl Amplifikationsgemisch und 5 µl Reaktionsansatz der Reversen Transkriptionsreaktion bzw. entsprechend gepufferter Zellprobe gebildet, wobei das Amplifikationsgemisch wie folgt zusammengesetzt ist:

| | |
|---|---|
| Puffer B (10 x) | 2 µl |
| 10 x BSA | 2 µl |
| Deoxynukleotidtriphosphate (dNTP's, 25 mM) | 1 µl |
| Amplimer | 0.5 µl |
| Taq Polymerase | 0.4 µl |
| H₂O | 14.3 µl |

Puffer B (10 x) ist wie folgt zusammengesetzt:

| | |
|---|---|
| 70 mM | MgCl₂ |
| 500 mM | KCl |
| 100 mM | β-mercaptoethanol |

Die Taq Polymerase (Biores) hat 5 U/µl (Andere Bestandteile siehe Beispiel 2).

Man gab nun 20 µl auf 89°C temperiertes Amplifikationsgemisch zu 5 µl Probe, die ebenfalls bei 89°C inkubiert wurde. Die DNA-Amplifikation wurde nun in 1-minütigen Zyklusschritten bei 89°C/63°C durchgeführt, wobei 25 bis 40 Zyklen in der Regel ausreichen, um HPV DNA bzw. mRNA in bis zu wenigstens 20 Zellen nachzuweisen.

### Beispiel 4a: Auswahl der Amplimere für HPV 18

1 ATTAATACTT TTAACAATTG TAGTATATAA AAAAGGGAGT AACCGAAAAC
51 GGTCGGGACC GAAAACGGTG TATATAAAAG ATGTGAGAAA CACACCACAA
101 TACTATGGCG CGCTTTGAGG ATCCAACACG GCGACCCTAC AAGCTACCTG E6
151 ATCTGTGCAC GGAACTGAAC ACTTCACTGC AAGACATAGA AATAACCTGT
201 GTATATTGCA AGACAGTATT GGAACTTACA GAGGTATTTG AATTTGCATT
251 TAAAGATTTA TTTGTGGTGT ATAGAGACAG TATACCCCAT GCTGCATGCC
301 ATAAATGTAT AGATTTTTAT GCTAGAATTA GAGAATTAAG ACATTATTCA
351 GACTCTGTGT ATGGAGACAC ATTGGAAAAA CTAACTAACA CTGGGTTATA
401 CAATTTATTA ATAAGGTGCC TGCGGTGCCA GAAACCGTTG AATCCAGCAG
451 AAAAACTTAG ACACCTTAAT GAAAAACGAC GATTTCACAA CATAGCTGGG
501 CACTATAGAG GCCAGTGCCA TTCGTGCTGC AACCGAGCAC GACAGGAACG
551 ACTCCAACGA CGCAGAGAAA CACAAGTATA ATATTAAGTA TGCATGGACC E7
601 TAAGGCAACA TTGCAAGACA TTGTATTGCA TTTAGAGCCC CAAAATGAAA
651 TTCCGGTTGA CCTTCTATGT CACGAGCAAT TAAGCGACTC AGAGGAAGAA
701 AACGATGAAA TAGATGGAGT TAATCATCAA CATTTACCAG CCCGACGAGC
751 CGAACCACAA CGTCACACAA TGTTGTGTAT GTGTTGTAAG TGTGAAGCCA
801 GAATTGAGCT AGTAGTAGAA AGCTCAGCAG ACGACCTTCG AGCATTCCAG
851 CAGCTGTTTC TGAACACCCT GTCCTTTGTG TGTCCGTGGT GTGCATCCCA
901 GCAGTAAGCA ACAATGGCTG ATCCAGAAGG TACAGACGGG GAGGGCACGG
951 GTTGTAACGG CTGGTTTTAT GTACAAGCTA TTGTAGACAA AAAAACAGGA

Amplimer-Positionen sind unterstrichen.
E6*-Spleißsequenzen sind gepunktet und unterstrichen.

Amplimer-Sequenzen:

Nr. 24 (Position 167 - 186)
AGT GAA TTC TTC GAA CAC TTC ACT GCA AGA CA

Nr. 26 (Position 667 - 686)
AGT GAA TTC GCG CGC TTA ATT GCT CGT GAC AT

Nr. 25 (Position 647 - 666)
AGT GAA TTC TCT AGA AGG TCA ACC GGA ATT TC

Die 4 Tripletts "Vorspann" sind EcoRI-spaltbare Oligonukleotidlinker.

Ein Intron befindet sich zwischen Position 236 bis 417 (182 bp), so daß bei Amplifikation der DNA mit dem Amplimerenpaar Nr.24 / Nr.26 eine Bande von 544 bp nachweisbar ist.

Bei Amplifikation der RNA, also nach Vorschaltung eines Schritts mit reverser Transkription der gespleißten mRNA, wird zusätzlich eine Bande von 362 bp nachgewiesen.

### Beispiel 4b: Auswahl der Amplimere für HPV 16

1 ACTACAATAA TTCATGTATA AAACTAAGGG CGTAACCGAA ATCGGTTGAA
51 CCGAAACCGG TTAGTATAAA AGCAGACATT TTATGCACCA AAAGAGAACT
101 GCAATGTTTC AGGACCCACA GGAGCGACCC AGAAAGTTAC CACAGTTATG E6
151 CACAGAGCTG CAAACAACTA TACATGATAT AATATTAGAA TGTGTGTACT
201 GCAAGCAACA GTTACTGCGA CGTGAGGTAT ATGACTTTGC TTTTCGGGAT
251 TTATGCATAG TATATAGAGA TGGGAATCCA TATGCTGTAT GTGATAAATG
301 TTTAAAGTTT TATTCTAAAA TTAGTGAGTA TAGACATTAT TGTTATAGTT
351 TGTATGGAAC AACATTAGAA CAGCAATACA ACAAACCGTT GTGTGATTTG
401 TTAATTAGGT GTATTAACTG TCAAAAGCCA CTGTGTCCTG AAGAAAAGCA
451 AAGACATCTG GACAAAAAGC AAAGATTCCA TAATATAAGG GGTCGGTGGA
501 CCGGTCGATG TATGTCTTGT TGCAGATCAT CAAGAACACG TAGAGAAACC
551 CAGCTGTAAT CATGCATGGA GATACACCTA CATTGCATGA ATATATGTTA E7
601 GATTTGCAAC CAGAGACAAC TGATCTCTAC TGTTATGAGC AATTAAATGA
651 CAGCTCAGAG GAGGAGGATG AAATAGATGG TCCAGCTGGA CAAGCAGAAC
701 CGGACAGAGC CCATTACAAT ATTGTAACCT TTTGTTGCAA GTGTGACTCT
751 ACGCTTCGGT TGTGCGTACA AAGCACACAC GTAGACATTC GTACTTTGGA
801 AGACCTGTTA ATGGGCACAC TAGGAATTGT GTGCCCCATC TGTTCTCAGA
851 AACCATAATC TACCATGGCT GATCCTGCAG GTACCAATGG GGAAGAGGGT
901 ACGGGATGTA ATGGATGGTT TTATGTAGAG GCTGTAGTGG AAAAAAAAAC
951 AGGGGATGCT ATATCAGATG ACGAGAACGA AAATGACAGT GATACAGGTG

Amplimer-Positionen sind unterstrichen.
E6^{*}-Spleißsequenzen sind gepunktet und unterstrichen.

Amplimer-Sequenzen:

Nr. 21 (Position 198 - 207)
AGT GAA TTC AGT ACT GCA AGC AAC AGT TAC TG

Nr. 22 (Position 601 - 620)
AGT GAA TTC AAC GTT GTC TCT GGT TGC AAA TC

Nr. 23 (Position 658 - 677)
AGT GAA TTC AGA TCT ATT TCA TCC TCC TCC TC

Die 4 Tripletts "Vorspann" jeweils sind EcoRI-spaltbare Oligonukleotidlinker

Die Introns befinden sich etwa bei Position 225 bis 410 (186 bp) und bei 254 bis 524 (270 bp). Bei Amplifikation der DNA mit dem Amplimerenpaar Nr.21 / Nr. 22 wird eine Bande von 447 bp nachweisbar, bei Amplifikation der RNA, also nach Vorschaltung eines Schritts mit reverser Transkription der gespleißten mRNA, werden zusätzlich Banden von etwa 261 und 177 bp nachgewiesen.
- Legende zu Fig. 1:: Offene Leserahmen von HPV 18 (Ähnlich ist HPV 16 aufgebaut).
- Legende zu Fig. 2:: Graphische Darstellung der E6/E7 Region von HPV 18
a) DNA-Organisation
b) E6/E7 mRNA von 3.4 kb
c) gespleißte mRNA für E6* von 1.6 kb
- Legende zu Fig. 3:: Graphische Darstellung der E6/E7 Region von HPV 16
a) DNA-Organisation
b) E6/E7 mRNA von 4.5 kb
c) gespleißte mRNA für E6* von 2.3 kb
d) kleinere gespleißte mRNA von etwa 1.5 kb
- Legende zu Fig. 4:: Amplimer-Positionen und Spleiß-Stellen in amplifizierten HPV 18 DNA Segmenten
a) E6/E7 Teil des HPV 18-Genoms
b) Position der Amplimere 24, 25 und 26.
c) Größe des amplifizierten DNA-Stücks bzw. der unprozessierten mRNA.
d) Größe der amplifizierten cDNA der gespleißten E6* RNA.
- Legende zu Fig. 5:: Amplimer-Positionen und Spleiß-Stellen in amplifizierten Segmenten von HPV 16 DNA
a) E6/E7 Teil des HPV 16-Genoms
b) Position der Amplimere 21, 22 und 23.
c) Größe des amplifizierten DNA-Stücks bzw. der unprozessierten mRNA.
d) Größe der amplifizierten cDNA der gespleißten E6* RNA
e) Größe der kleineren mRNA Bande, die durch Smotkin u. Wettstein, Proc. Natl. Acad. Sci (USA) 83, 4680 (1986) identifiziert wurde.

## Patentansprüche (Patentansprüche für folgende(n) Vertragsstaat(en): AT, BE, CH, DE, FR, GB, IT, LI, LU, NL, SE)

1. Verfahren zum Nachweis gespleißter mRNA, dadurch gekennzeichnet, daß
a) ein cDNA-Syntheseschritt mit reverser Transskriptase und
b) eine PCR unter Verwendung von Amplimeren, welche eine Spleißregion überspannen,
nacheinander durchgeführt werden.

2. Verfahren nach Anspruch 1, dadurch gekennzeichnet, daß die Amplimere eine Spleißregion der HPV 16 oder HPV 18 E6-Region überspannen.

3. Verfahren nach Anspruch 1, dadurch gekennzeichnet, daß die nachzuweisenden mRNA durch Beschallung aus einer Zellprobe freigesetzt wird.

4. Verfahren nach Anspruch 1, dadurch gekennzeichnet, daß die PCR bei Zugabe von bis zu 20 % v/v Dimethylsulfoxid durchgeführt wird.

5. Verfahren nach Anspruch 1, dadurch gekennzeichent, daß die PCR
a) unter Einsatz von Deoxynukleotidtriphosphatkonzentrationen > 0,2 mM und Amplimerkonzentrationen > 0,5 µM
b) mit Zykluszeiten von 1-2 Minuten
c) bei 2 Temperaturen von 70 °C bis 95 °C und 50 °C bis 70 °C
d) nach Start der PCR oberhalb der Schmelztemperatur
e) mit direktem Nachweis spezifischer DNA-Fragmente durch Färbung nach Agarosegelelektrophorese
durchgeführt wird.

6. Verfahren nach Anspruch 5, dadurch gekennzeichent, daß die Temperaturen 89 °C und 63 °C gewählt werden.

7. Verfahren zum Nachweis prämaligner oder maligner Zustände oder Läsionen durch den direkten Nachweis gespleißter HPV-spezifischer mRNA gemäß Anspruch 2.

## Patentansprüche (Patentansprüche für folgende(n) Vertragsstaat(en): ES)

1. Verfahren zum Nachweis gespleißter mRNA, dadurch gekennzeichnet, daß
a) ein cDNA-Syntheseschritt mit reverser Transskriptase und
b) eine PCR unter Verwendung von Amplimeren, welche eine Spleißregion überspannen,
nacheinander durchgeführt werden.

2. Verfahren nach Anspruch 1, dadurch gekennzeichnet, daß die Amplimere eine Spleißregion der HPV 16 oder HPV 18 E6-Region überspannen.

3. Verfahren nach Anspruch 1, dadurch gekennzeichnet, daß die nachzuweisenden mRNA durch Beschallung aus einer Zellprobe freigesetzt wird.

4. Verfahren nach Anspruch 1, dadurch gekennzeichnet, daß die PCR bei Zugabe von bis zu 20 % v/v Dimethylsulfoxid durchgeführt wird.

5. Verfahren nach Anspruch 1, dadurch gekennzeichent, daß die PCR
a) unter Einsatz von Deoxynukleotidtriphosphatkonzentrationen > 0,2 mM und Amplimerkonzentrationen > 0,5 µM
b) mit Zykluszeiten von 1-2 Minuten
c) bei 2 Temperaturen von 70 °C bis 95 °C und 50 °C bis 70 °C
d) nach Start der PCR oberhalb der Schmelztemperatur
e) mit direktem Nachweis spezifischer DNA-Fragmente durch Färbung nach Agarosegelelektrophorese
durchgeführt wird.

6. Verfahren nach Anspruch 5, dadurch gekennzeichent, daß die Temperaturen 89 °C und 63 °C gewählt werden.

7. Verfahren zum Nachweis prämaligner oder maligner Zustände oder Läsionen durch den direkten Nachweis gespleißter HPV-spezifischer mRNA gemäß Anspruch 2.

## Claims (Claims for the following Contracting State(s): AT, BE, CH, DE, FR, GB, IT, LI, LU, NL, SE)

1. A method for detecting spliced mRNA, which comprises successively carrying out
a) a cDNA synthesis step using reverse transcriptase, and
b) a PCR using amplimers which span a splicing region.

2. The method as claimed in claim 1, wherein the amplimers span a splicing region of the HPV 16 or HPV 18 E6 region.

3. The method as claimed in claim 1, wherein the mRNA to be detected is liberated from a cell sample by sonication.

4. The method as claimed in claim 1, wherein the PCR is carried out with the addition of up to 20 % v/v dimethyl sulfoxide.

5. The method as claimed in claim 1, wherein the PCR is carried out
a) using deoxynucleotide triphosphate concentrations of > 0.2 mM and amplimer concentrations of > 0.5 µM
b) using cycle times of 1-2 minutes
c) at 2 temperatures of 70°C to 95°C and of 50°C to 70°C
d) after starting the PCR above the melting temperature
e) with the direct detection of specific DNA fragments by staining after agarose gel electrophoresis.

6. The method as claimed in claim 5, wherein the temperatures of 89°C and 63°C are chosen.

7. A method for detecting premalignant or malignant conditions or lesions by means of directly detecting spliced HPV-specific mRNA as claimed in claim 2.

## Claims (Claims for the following Contracting State(s): ES)

1. A method for detecting spliced mRNA, which comprises successively carrying out
a) a cDNA synthesis step using reverse transcriptase, and
b) a PCR using amplimers which span a splicing region.

2. The method as claimed in claim 1, wherein the amplimers span a splicing region of the HPV 16 or HPV 18 E6 region.

3. The method as claimed in claim 1, wherein the mRNA to be detected is liberated from a cell sample by sonication.

4. The method as claimed in claim 1, wherein the PCR is carried out with the addition of up to 20 % v/v dimethyl sulfoxide.

5. The method as claimed in claim 1, wherein the PCR is carried out
a) using deoxynucleotide triphosphate concentrations of > 0.2 mM and amplimer concentrations of > 0.5 µM
b) using cycle times of 1-2 minutes
c) at 2 temperatures of 70°C to 95°C and of 50°C to 70°C
d) after starting the PCR above the melting temperature
e) with the direct detection of specific DNA fragments by staining after agarose gel electrophoresis.

6. The method as claimed in claim 5, wherein the temperatures of 89°C and 63°C are chosen.

7. A method for detecting premalignant or malignant conditions or lesions by means of directly detecting spliced HPV-specific mRNA as claimed in claim 2.

## Revendications (Revendications pour l'(les) Etat(s) contractant(s) suivant(s): AT, BE, CH, DE, FR, GB, IT, LT, LU, NL, SE)

1. Procédé pour la détection d'ARNm épissé, caractérisé en ce que l'on effectue successivement
a) une étape de synthèse d'ADNc à l'aide de transcriptase inverse et
b) une PCR (réaction de polymérisation en chaîne) en utilisant des amplimères qui recouvrent une région d'épissage.

2. Procédé selon la revendication 1, caractérisé en ce que les amplimères recouvrent une région d'épissage de la région E6 de HPV 16 ou HPV 18.

3. Procédé selon la revendication 1, caractérisé en ce que l'ARNm à détecter est libéré par sonication à partir d'un échantillon cellulaire.

4. Procédé selon la revendication 1, caractérisé en ce que la PCR est effectuée avec addition de jusqu'à 20 % v/v de diméthylsulfoxyde.

5. Procédé selon la revendication 1, caractérisé en ce que la PCR est effectuée
a) avec utilisation de concentrations de désoxynucléotide triphosphates > 0,2 mM et des concentrations d'amplimères > 0,5 µM,
b) avec des temps de cycle de 1-2 minutes,
c) à deux températures de 70 à 95°C et de 50 à 70°C,
d) après le début de la PCR, au-dessus de la température de fusion,
e) avec détection directe de fragments d'ADN spécifiques par coloration après électrophorèse en gel d'agarose.

6. Procédé selon la revendication 5, caractérisé en ce que l'on choisit les températures de 89 et 63°C.

7. Procédé pour la mise en évidence de lésions ou d'états prémalins ou malins, par la détection directe d'ARNm épissé spécifique de HPV selon la revendication 2.

## Revendications (Revendications pour l'(les) Etat(s) contractant(s) suivant(s): ES)

1. Procédé pour la détection d'ARNm épissé, caractérisé en ce que l'on effectue successivement
a) une étape de synthèse d'ADNc à l'aide de transcriptase inverse et
b) une PCR (réaction de polymérisation en chaîne) en utilisant des amplimères qui recouvrent une région d'épissage.

2. Procédé selon la revendication 1, caractérisé en ce que les amplimères recouvrent une région d'épissage de la région E6 de HPV 16 ou HPV 18.

3. Procédé selon la revendication 1, caractérisé en ce que l'ARNm à détecter est libéré par sonication à partir d'un échantillon cellulaire.

4. Procédé selon la revendication 1, caractérisé en ce que la PCR est effectuée avec addition de jusqu'à 20 % v/v de diméthylsulfoxyde.

5. Procédé selon la revendication 1, caractérisé en ce que la PCR est effectuée
a) avec utilisation de concentrations de désoxynucléotide triphosphates > 0,2 mM et des concentrations d'amplimères > 0,5 µM,
b) avec des temps de cycle de 1-2 minutes,
c) à deux températures de 70 à 95°C et de 50 à 70°C,
d) après le début de la PCR, au-dessus de la température de fusion,
e) avec détection directe de fragments d'ADN spécifiques par coloration après électrophorèse en gel d'agarose.

6. Procédé selon la revendication 5, caractérisé en ce que l'on choisit les températures de 89 et 63°C.

7. Procédé pour la mise en évidence de lésions ou d'états prémalins ou malins, par la détection directe d'ARNm épissé spécifique de HPV selon la revendication 2.
